# EUROPÄISCHE PATENTANMELDUNG

(11) **EP 0 611 024 A2**
(43) Veröffentlichungstag der Anmeldung: **17.08.1994**
(21) Anmeldenummer: 94250014.1
(22) Anmeldetag: 27.01.1994
(51) Int. Cl.: B01F 17/00, C11D 3/38, C12N 1/00

(54) **Grenzflächenchemisch aktive Verbindungen aus mikrobiellen Biomassen**

(30) Priorität: 10.02.1993 DE 4304550
(71) Anmelder: AUF ANALYTIK UMWELTTECHNIK FORSCHUNG GmbH, D-12484 Berlin (DE)
(72) Erfinder: Olschewski, Max, D-12557 Berlin (DE); Olschewski, Brigitte, Dr., D-12557 Berlin (DE)
(74) Vertreter: Haupt, Wolfgang J.

(57) **Zusammenfassung**

Die Erfindung betrifft eine Mischung grenzflächenchemisch aktiver Verbindungen, die durch Reaktion von mikrobiellen Biomassen mit Alkylhalogeniden, Epoxiden oder Säurehalogeniden erhalten werden, wobei die Reaktion in Anwesenheit eines katalytisch wirksamen Mittels und eines oder mehrerer mit Wasser nicht mischbarer organischer Lösungsmittel durchgeführt wird.

Diese Verbindungen sind als im wesentlichen nichtionische Tenside mit leichtem anionischen Anteil beispielsweise als Schäumungsmittel, Reinigungsmittel, Emulgatoren für die unterschiedlichsten organischen Stoffe in Wasser und für Wasser-in-Öl-Emulsionen sowie im weitesten Sinne als Netzmittel verwendbar.

## Beschreibung

Die Erfindung betrifft eine Mischung grenzflächenchemisch aktiver Verbindungen, die aus mikrobiellen Biomassen erhalten werden.
Diese Verbindungen sind als im wesentlichen nichtionische Tenside mit leichtem anionischen Anteil beispielsweise als Schäumungsmittel, Reinigungsmittel, Emulgatoren für die unterschiedlichsten organischen Stoffe in Wasser und für Wasser-in-Öl-Emulsionen sowie im weitesten Sinne als Netzmittel verwendbar.

Aufgrund günstiger Kosten-Nutzen-Relationen sind vor allem folgende Tenside in hoher Produktmenge auf den Markt gekommen:
Alkansulfonate, lineare Alkylbenzolsulfonate, Alkylethersulfate, Alkoholethoxylate, Alkylsulfate und Seife. Einige von ihnen, z.B. Alkylbenzolsulfate, sollten wegen ihren negativen ökologischen Eigenschaften substituiert werden.
Im Zeitalter einer zunehmenden Verknappung fossiler Rohstoffe und steigendem Umweltbewußtsein liegt der Schwerpunkt der Tensidchemie heute auf der Synthese von umweltverträglichen Tensiden auf Basis nachwachsender Rohstoffe [B.Fabry, Chemie in unserer Zeit 25 (1991) Nr.4]. Dabei zeichnen sich hinsichtlich eines guten Eigenschaftsbildes und günstigen ökologischen Vergleiches zwei wesentliche Tensidklassen ab, nämlich die Alkylpolyglucoside und die Eiweiß-Fettsäure-Kondensationsprodukte.
Zur Herstellung von Polyglucosiden werden entweder Saccharidhydroxylgruppen mit Fettalkoholen sauer veräthert (DE-40 06 192 und EP-448 799), Saccharide mit Fettsäuren oder Fettsäurechloriden verestert (EP-268 974) bzw. darüber hinaus noch oligoalkoxyliert (DE-41 03 681 und EP-405 967) oder auch Aldehydgruppen der Saccharide durch reduzierende Aminierung und anschließende Reaktion der Aminogruppen mit Säurechlorid zu Amiden umgesetzt (DE-35 38 451). Außerdem zeigen Zusätze solcher Polyglucoside zu Alkylethersulfat oder Alkansulfonat synergistische Effekte (DE-40 19 790). Alkylpolyglucoside besitzen interessante anwendungstechnische Eigenschaften wie synergistische Wechselwirkungen mit vielen wichtigen Tensiden, höhere Schaumaktivität als übliche nichtionische Tenside, verbesserte Hautverträglichkeit und gute biologische Abbaubarkeit.

Eine weitere Gruppe der milden Tenside mit guter Haut- und Schleimhautverträglichkeit sind die Eiweiß-Fettsäure-Kondensationsprodukte. Diese werden mit den normalen wasch- und reinigungsaktiven Mitteln kombiniert, um die Hautverträglichkeit der Alkylsulfate, α-Olefinsulfonate und Alkylbetaine zu verbessern. Dazu werden beispielsweise Eiweißhydrolysate der Molmasse 3000 bis 7000 mit Säurechlorid (EP-417 619 und EP-379 101) oder auch mit Fettsäureestern (DD-273 275) kondensiert. Die Eiweiß-Fettsäure-Kondensate werden immer in Kombination mit anderen Tensiden eingesetzt. Ihre Oberflächenspannung ist auch bei hohen Konzentrationen ≧ 30 mN/m. Die zu ihrer Herstellung dienenden Eiweißhydrolysate sind aus pflanzlichen und tierischen Proteinen erhältlich.
Die vorteilhaften Eigenschaften dieser Produkte bestehen in der fehlenden Toxizität, der reizhemmenden Wirkung in Kombination mit üblichen Tensiden und in einer guten Kompatibilität mit anionischen, nichtionischen sowie teilweise mit quaternären Ammoniumverbindungen. Sie besitzen selbst eine milde Reinigungswirkung.

Die Erfindung bezweckt die Herstellung grenzflächenchemisch aktiver Verbindungen mit einer Oberflächenspannung der wäßrigen Lösung von ≦ 30 mN/m, die damit in besonderer Weise zur Benetzung von zahlreichen, unterschiedlichen polaren und unpolaren, aliphatischen und aromatischen Stoffen geeignet sind.

Es bestand dabei die Aufgabe, mikrobielle Biomassen, die zahlreiche und unterschiedliche reaktionsfähige Gruppen enthalten und die von Natur aus weder wasserlöslich sind noch nennenswerte grenzflächenchemische Eigenschaften aufweisen, in einfacher und ökonomisch günstiger Art und Weise durch geeignete Derivatisierung in grenzflächenaktive Stoffe zu überführen.

Erfindungsgemäß wird aus einer mikrobiellen Biomasse durch Derivatisierung ihrer reaktionsfähigen Gruppen wie beispielsweise deren Umsetzung zu Ethern, Alkoxyethern, Estern, Phosphorsäureestern, Amiden, Imiden, Betainen, u.dgl. sowie deren Gemischen eine Mischung grenzflächenchemisch aktiver Verbindungen hergestellt.

Geeignete mikrobielle Biomassen sind z.B. Hefen wie Bäckerhefe, Brauhefe, Gärhefe in frischem Zustand wie auch als Abfallbiomasse oder Bakterien, z.B. in Form von Belebtschlamm. Ein möglicher, aber nicht notwendiger, partieller Aufschluß der mikrobiellen Biomasse vor der Derivatisierung kann entweder im ameisensauren oder alkalischen Medium erfolgen, wobei enthaltene Ester und Amide ggfl. hydrolysiert werden.
Etherderivate können günstigerweise durch Reaktion in einem inerten Lösungsmittel mit Alkylhalogenid in Gegenwart von Hydrogencarbonat erhalten werden.
Alkoxyetherderivate werden z.B. durch Umsetzung im alkalischen Medium in inerten Lösungsmitteln mit Propylenoxid, Ethylenoxid u.a. unter erhöhtem oder Normaldruck und erhöhter Temperatur erhalten.
Amide und Imide entstehen z.B. durch Reaktion der primären und sekundären Amine aus den Eiweißbestandteilen der Biomassen mit einem Fettsäurehalogenid.
Esterderivate werden vorteilhafterweise durch Umsetzung der Hydroxylgruppen der Polysaccharidanteile mit Säurehalogenid im alkalischen Medium hergestellt.
Die Reaktion wird in Anwesenheit eines oder mehrerer mit Wasser nicht mischbarer Lösungsmittel durchgeführt, die gegenüber den Reaktionspartnern inert sind. Toluol ist hierbei bevorzugt.

Als katalytisch wirksame Mittel können Alkalimetallhydroxide, -carbonate oder -hydrogencarbonate eingesetzt werden.
Als besonders vorteilhaft ist die Tatsache hervorzuheben, daß die für das erfindungsgemäße Verfahren verwendeten mikrobiellen Biomassen im wesentlichen vorher nicht aufgeschlossen werden müssen.

Weiterhin ist aufgrund der sehr zahlreichen Kombinationsmöglichkeiten unterschiedlicher Derivatisierung in quantitativer und qualitativer Hinsicht eine sehr hohe Anpassungsfähigkeit der grenzflächenchemischen Eigenschaften an den jeweiligen gewünschten Einsatzzweck gegeben.

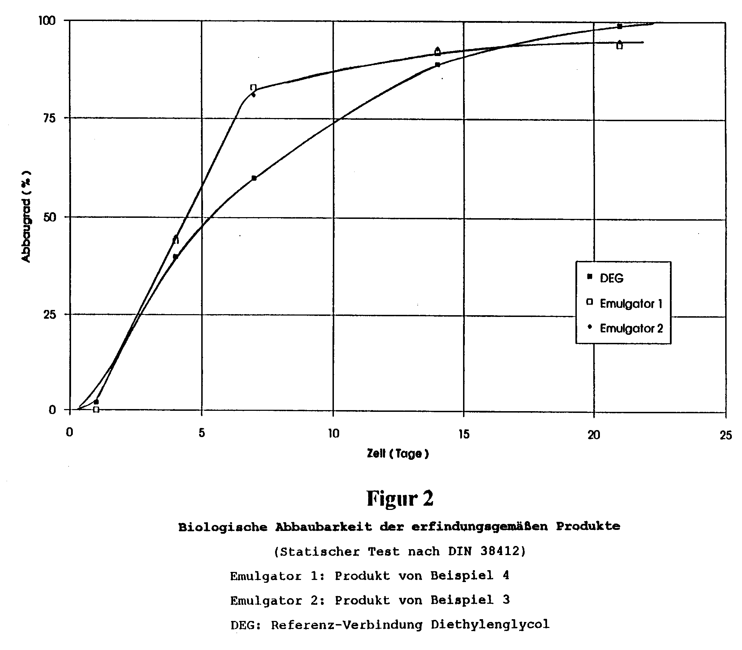

Außerdem ist hervorzuheben, daß die erfindungsgemäß erhaltenen Produkte eine ausgezeichnete biologische Abbaubarkeit aufweisen (vgl. Fig. 2). Wie aus der Darstellung zu sehen ist, sind die zwei untersuchten Produkte bereits nach 20 Tagen zu etwa 90 % abgebaut.
Die Erfindung wir nachstehend ohne einschränkenden Charakter an Ausführungsbeispielen näher erläutert.

### BEISPIEL 1

333 g Gärhefe (30 %ig) mit einem Stickstoffgehalt von 8,7 % werden mit 100 g 40 %iger Natronlauge versetzt, 3 Stunden lang bei einer Temperatur von 85 °C belassen und dabei 150 g Wasser unter leichtem Vakuum abgezogen. Nach dem Abkühlen auf etwa 40 °C werden jeweils innerhalb von 5 Minuten nacheinander 30 g Stearinsäurechlorid in 200 ml Toluol und 20 g Caprylsäurechlorid in 20 ml Toluol hinzugegeben und anschließend noch 1 Stunde lang bei 80 °C gerührt.
Danach werden 100 ml Toluol zugesetzt und das gesamte Wasser unter leichtem Wasserstrahlvakuum im Wasserabscheider entfernt. Anschließend gibt man bei einer Temperatur von 100 °C innerhalb von 2 Stunden tropfenweise 50 g Ethylenoxid hinzu (Trockeneis-Methanol-Kühlung). Dann wird, wie bereits in Beispiel 1 beschrieben, das Toluol durch gleichzeitige Zugabe von 400 bis 500 ml Wasser entfernt.
Man erhält eine leicht bräunliche, viskose Flüssigkeit mit einem pH-Wert von 10 und einem Feststoffgehalt von 35 %.

### BEISPIEL 2

500 g Klärschlamm (20 %ig) mit einem Stickstoffgehalt von 7,8 % werden mit 100 g 40 %iger Natronlauge versetzt, 4 Stunden lang bei einer Temperatur von 90 °C belassen und dabei 150 g Wasser unter leichtem Vakuum abgezogen. Nach dem Abkühlen auf etwa 40 °C werden innerhalb von 5 Minuten 60 g Palmitinsäurechlorid in 200 ml Toluol hinzugegeben und anschließend noch 1 Stunde lang bei 80 °C gerührt.
Danach werden 100 ml Toluol zugesetzt und das gesamte Wasser unter leichtem Wasserstrahlvakuum im Wasserabscheider entfernt. Anschließend gibt man bei einer Temperatur von 100 °C innerhalb von 2 Stunden tropfenweise 44 g Ethylenoxid hinzu (Trockeneis-Methanol-Kühlung). Dann wird, wie bereits in Beispiel 1 beschrieben, das Toluol durch gleichzeitige Zugabe von 400 bis 500 ml Wasser entfernt.
Man erhält eine leicht bräunliche, viskose Flüssigkeit mit einem pH-Wert von 10,5 und einem Feststoffgehalt von 26,2 %.

### BEISPIEL 3

100 g wirbelsintergetrocknete Bäckerhefe mit einem Stickstoffgehalt von 6,7 % werden mit 100 g 40 %iger Natronlauge versetzt und 1 Stunde lang bei einer Temperatur von 70 °C gerührt. Nach dem Abkühlen auf etwa 40 °C werden 50 g Laurinsäurechlorid, gelöst in 200 ml Toluol, innerhalb von 10 Minuten tropfenweise hinzugegeben und danach weitere 30 Minuten lang bei 80 °C bis zur Vervollständigung der Reaktion gerührt. Nach dem Abkühlen auf eine Temperatur von etwa 40 °C werden 40 g Laurylchlorid, gelöst in 150 ml Toluol, innerhalb von 10 Minuten dazugetropft. Nach weiterem Rühren 3 Stunden lang bei 80 °C werden etwa 250 ml Toluol unter leichtem Wasserstrahlvakuum abdestilliert, anschließend nach und nach etwa 400 ml Wasser zugegeben und im gleichen Maße das restliche Toluol azeotrop unter leichtem Wasserstrahlvakuum entfernt.
Man erhält eine leicht bräunliche, viskose Flüssigkeit mit einem pH-Wert von 9,5 und einem Feststoffgehalt von 28,5 %.

### BEISPIEL 4

400 g Abfall-Brauhefe (25 %ig) mit einem Stickstoffgehalt von 7,9 % (bezogen auf Trockensubstanz) werden mit 240 g 40 %iger Natriumcarbonat-Losung versetzt, 1 Stunde lang bei einer Temperatur von 90 °C gerührt und dabei unter leichtem Wasserstrahlvakuum 200 g Wasser abgezogen. Nach dem Abkühlen auf etwa 40 °C werden jeweils innerhalb von 10 Minuten nacheinander 50 g Palmitinsäurechlorid in 100 ml Toluol, 50 g Myristinsäurechlorid in 100 ml Toluol sowie 30 g Caprinsäurechlorid in 60 ml Toluol zugegeben.
Anschließend wird 1 Stunde lang bei 95 °C gerührt und die Mischung danach wie in Beispiel 1 aufgearbeitet.
Man erhält ein leicht pastöses Produkt mit einem pH-Wert von 8,7 und einem Feststoffgehalt von 25 %.

### BEISPIEL 5

Man verfährt wie in Beispiel 1, jedoch ausgehend von 400 g Brauhefe (25 %) mit einem Stickstoffgehalt von 7,9 %, 100 g 40 %iger Natronlauge, 50 g Caprylbromid, 25 g Laurinsäurechlorid und 20 g Monochloressigsäure sowie einer Vorreaktionsdauer von 1 Stunde bei 90 °C.
Man erhält eine leicht bräunliche, viskose Flüssigkeit mit einem pH-Wert von 8,7 und einem Feststoffgehalt von 33,0 %.

### BEISPIEL 6

Man verfährt wie in Beispiel 1, jedoch ausgehend von 500 g Klärschlamm (20 %) mit einem Stickstoffgehalt von 7,8 %, 100 g 40 %iger Natronlauge, 40 g Laurylbromid, 80 g Laurinsäurechlorid und 20 g Ethylenoxid sowie einer Vorreaktionsdauer von 5 Stunden bei 90 °C.
Man erhält eine leicht bräunliche, viskose Flüssigkeit mit einem pH-Wert von 9,3 und einem Feststoffgehalt von 22,8 %.

Die nachfolgende Tabelle I zeigt in einer Übersicht die Tensid-Eigenschaften der erhaltenen Produkte.

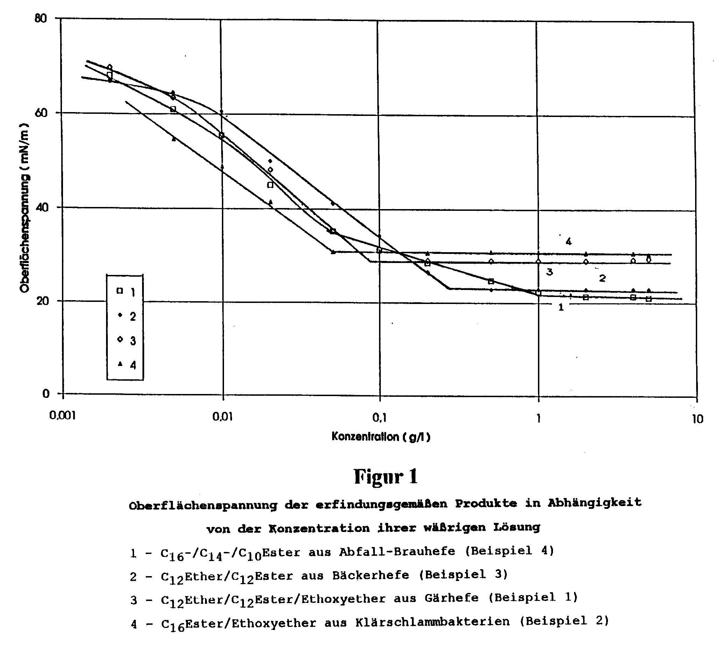

Die Figur 1 zeigt in einer Kurvendarstellung die Oberflächenspannung der erfindungsgemäßen Produkte in Abhängigkeit von der Konzentration ihrer wäßrigen Lösung.
In Figur 2 ist die biologische Abbaubarkeit der Produkte dargestellt (statischer Test nach DIN 38412).

**TABELLE I**

| Tensid-Eigenschaften der Produkte | | | | | |
|---|---|---|---|---|---|
| Beispiel | Oberfl.-Spannung | | SCHAUM-Aktivität Stabilität (Ross-Miles, DIN *) | pH-Wert | Feststoff-Gehalt (%) |
| | σ [mN/m] | CMC [g/l] | | | |
| 1 (K) | 24,8 | 7·10⁻² | 195/130 | 10,0 | 35,0 |
| 2 (K) | 31,0 | 5·10⁻² | 135/130 | 10,5 | 26,2 |
| 3 (K) | 29,5 | 8·10⁻² | 120/110 | 9,5 | 28,5 |
| 4 (K) | 21,1 | 1·10^{o} | 35/20 | 7,5 | 23,5 |
| 5 | 26,0 | 5·10⁻¹ | 70/70 | 8,7 | 33,0 |
| 6 | 22,9 | 3·10⁻¹ | 160/150 | 9,3 | 22,8 |

| | | | | | |
|---|---|---|---|---|---|
| (K) Kurvenbild in Figur 1 | | | | | |
| * DIN 53902 | | | | | |

## Patentansprüche

1. Mischung grenzflächenchemisch aktiver Verbindungen, dadurch gekennzeichnet, daß sie durch Reaktion von mikrobiellen Biomassen mit Alkylhalogeniden und/oder Epoxiden und/oder Säurehalogeniden erhalten werden, wobei die Reaktion in Anwesenheit eines katalytisch wirksamen Mittels und eines oder mehrerer mit Wasser nicht mischbarer organischer Lösungsmittel durchgeführt wird.

2. Mischung nach Anspruch 1, dadurch gekennzeichnet, daß die zu ihrer Herstellung verwendeten Alkylhalogenide und/oder Epoxide und/oder Säurehalogenide in ihren Alkylresten oder Alkylteilen 1 bis 20 Kohlenstoffatome in gerader oder verzweigter Kette aufweisen.

3. Mischung nach Anspruch 1 und 2, dadurch gekennzeichnet, daß die zu ihrer Herstellung verwendeten Alkylhalogenide und/oder Epoxide und/oder Säurehalogenide in bezug auf die Gesamtheit der funktionellen Gruppen in der mikrobiellen Biomasse in unterstöchiometrischen Mengen eingesetzt werden, um einen Derivatisierungsgrad von unter 1 zu erhalten.

4. Mischung nach Anspruch 1 bis 3, dadurch gekennzeichnet, daß als katalytisch wirksame Mittel Alkalimetallhydroxide, -carbonate oder -hydrogencarbonate eingesetzt werden.
